# EUROPEAN PATENT SPECIFICATION

(11) **EP 3 510 393 B1**
(45) Date of publication and mention of the grant of the patent: **04.11.2020**
(21) Application number: 17849433.2
(22) Date of filing: 06.09.2017
(51) Int. Cl.: C07B 59/00, G01N 31/22, G01N 27/447, G01N 30/88, A61K 51/04

(54) **METHOD AND DEVICE FOR CONCENTRATION AND FORMULATION OF RADIOPHARMACEUTICALS**
VERFAHREN UND VORRICHTUNG ZUR KONZENTRATION UND FORMULIERUNG VON RADIOPHARMAZEUTIKA
PROCÉDÉ ET DISPOSITIF POUR LA CONCENTRATION ET LA FORMULATION DE PRODUITS RADIOPHARMACEUTIQUES

(30) Priority: 06.09.2016 US 201662384084 P
(43) Date of publication of application: 17.07.2019
(73) Proprietor: The Regents of the University of California, Oakland, CA 94607 (US)
(72) Inventor: CHAO, Philip Hong-Sean, Los Angeles, CA 90034 (US); VAN DAM, R. Michael, Los Angeles, CA 90024 (US)
(74) Representative: Lecomte & Partners
(86) International application number: PCT/US2017/050236
(87) International publication number: WO 2018/048873

(56) References cited:
- US-A1- 2008 036 668
- US-A1- 2008 233 018
- US-A1- 2008 281 090
- US-A1- 2012 101 268
- US-A1- 2016 130 295

## Description

### Technical Field

The technical field generally relates to microfluidic methods and devices used for the concentration and formulation (or reformulation) of radiolabeled compounds.

### Background

Positron emission tomography (PET) uses tiny amounts of short-lived radiolabeled tracers to image specific molecular processes in living subjects. It is used in the clinic to diagnose disease, monitor disease progression and assess response to therapy in many fields including oncology, neurology, cardiology, immunology, infectious diseases, etc. PET can also be used in preclinical research to study fundamental disease processes, and the development of therapies. Following synthesis of most positron emission tomography (PET) tracers, purification is performed via semi-preparative high performance liquid chromatography (HPLC). Purified product is then collected in a mobile phase with volumes up to tens of milliliters. Since the mobile phase contains solvents such as acetonitrile (MeCN) or ethanol (EtOH), formulation involves removal of these solvents to acceptable levels. Formulation is performed using solid-phase extraction (SPE) and/or evaporation. In reversed-phase SPE, the sample is diluted with water, then passed through a cartridge (e.g., C18) to trap the tracer. After washing the cartridge, the tracer is then eluted with 1-2 mL of EtOH. Dilution with saline (10-20 mL) could make the probe unusable for preclinical imaging, e.g., mice, where the maximum injection volume is -100 µL, so the EtOH is often evaporated first and the dried tracer reconstituted in saline. While direct evaporation of the original sample is also possible, this requires bulky apparatus (e.g., rotary evaporator) to achieve desired evaporation rates and involves manual intervention.

Commercially available evaporation systems (rotary evaporators) have the ability to concentrate or formulate PET tracers by evaporation, but the systems are extremely bulky and take up a large amount of valuable space within radiation-shielded "hot cells." In addition, the rotary evaporation process is relatively slow and requires manual intervention to: introduce sample for concentration, assess the vacuum level to prevent splashing during concentration, assess completion of the evaporation process, introduce saline, and collect the formulated product. Due to these issues, rotary evaporation is not often used in practice, and formulation is instead done through alternative means such as solid phase extraction. More compact, commercial, rotary evaporation systems have recently been reported that use a vortex air stream paired with heating of the sample for evaporation and solvent removal. An example of such a system is the Smart Evaporator system that uses vacuum-assisted vortex concentration to increase the contact surface area between the liquid and gas which promotes evaporation, which is distributed by Eicom USA (San Diego, CA). These systems are significantly smaller than traditional rotovaps, but still much larger than the microfluidic chip described herein. The Smart Evaporator system, however, was found to have too low an evaporation rate (<∼0.3 mL/min), which is particularly problematic for time-sensitive applications such as concentration of short-lived radioactive tracers. Moreover, the system is not automatic and would require operator intervention.

Other microfluidic chip architectures have been reported for concentrating samples, however, these devices also have very low evaporation rates and would not be suitable for the concentration of PET tracers. While rapid evaporation was demonstrated, the ability to sufficiently remove solvents was not explored, and, in most cases, an additional formulation step (solvent exchange) was needed before concentration to ensure that the final concentrated buffer would be safe for injection.

Microfluidic devices can offer a variety of advantages over macroscopic reactors (K. Janish et al., Angew. Chem. 2004, 116:410-451, P. Watts et al., Chem. Soc. Rev. 2005, 34:235-246, G. Jas et al., Chem. Eur. J. 2003, 9:5708-5723).US 2008/0233018 A1 discloses microfluidic devices and chemical processes using such devices. More specifically, it is disclosed a fully automated synthesis of radioactive compounds for imaging such as by positron emission tomography (EPT). Radiolabeled compounds may be produced using macroscopic synthesizers (WO 2007/066089, WO2005/057589, US 2007/0031492 and US 2004/022696).

### Summary

The invention is directed to a method of formulating or concentrating radiolabeled molecule or compound, according to claim 1.

Further developments of the invention are according to dependent claims 2-9.

The invention is also directed to a system for formulating or concentrating radiolabeled molecule or compound, according to claim 10.

Further developments of the invention are according to dependent claims 11-14.

### Brief Description of the Drawings

FIG. 1 illustrates a perspective view of a microfluidic device or chip that is used as a microfluidic concentrator. FIG. 1 further illustrates the multi-layer construction including the compression layer, sample layer, permeably membrane, and gas flow layer.
FIG. 2A illustrates a cross-sectional view of the fully assembled microfluidic device or chip taken along the line A-A of FIG. 1.
FIG. 2B illustrates a cross-sectional view of the fully assembled microfluidic device or chip taken along the line B-B of FIG. 1.
FIG. 3 illustrates one embodiment of an automated system 60 that incorporates the microfluidic device 10 described herein.
FIG. 4 illustrates a series of illustrative operational steps used to concentrate a sample using the microfluidic device according to one embodiment. Alongside each operational step is a photographic image of the microfluidic device.
FIG. 5 is a graph showing the evaporation rates of deionized water as a function of temperature for different sample layer materials. Data points show an average of five (5) repeats and error bars represent standard deviation.
FIG. 6 illustrates a graph of sample recovery measurements (% recovery) using [¹⁸F]fluoride solutions. Results are shown for different sample layer materials. All data is for the partial evaporation method of operation, except PMMA-machined, for which both partial and complete evaporation modes were tested. Each data point represents n=3 repeats with error bars denoting standard deviation.
FIG. 7 illustrates a graph of sample recovery measurements (% recovery) using [¹⁸F]fluoride solutions enable in-chip measurement of the effects of breakthrough. The data indicate minimal loss of solute for mobile phases containing up to 80% MeCN v/v (in water), or up to 40% EtOH v/v (in water).
FIG. 8 illustrates a graph of percent recovery of different tracers on different sample layer materials concentrated with both partial and complete evaporation modes. Unless otherwise stated, each bar represents three repeats. (*) represents one repeat, (o) represents two repeats, (Δ) represents four repeats, (•) represents six repeats and (◇) represents nine repeats. Error bars represent standard deviation.
FIG. 9A illustrates contour plots of % residual solvent after concentration by partial evaporation of MeCN/water mixtures.
FIG. 9B illustrates contour plots of % residual solvent after concentration by partial evaporation of EtOH/water mixtures.

### Detailed Description of the Illustrated Embodiments

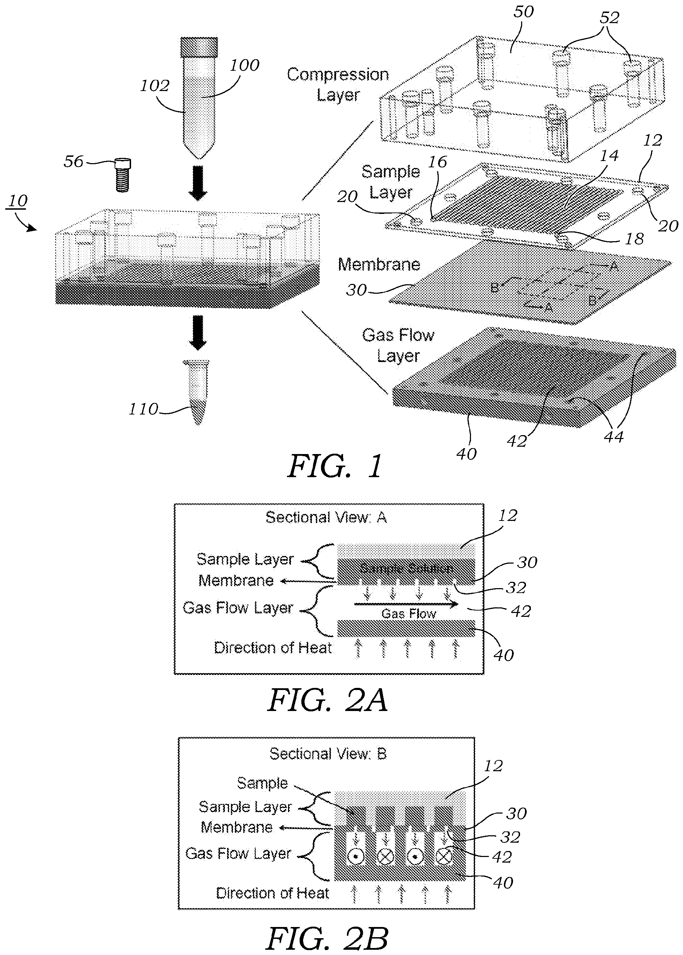

FIG. 1 illustrates a microfluidic device 10 or "chip" that is used as a microfluidic concentrator. In one particular embodiment, the microfluidic device 10 is used to concentrate a radiolabeled molecule or compound. As an example of such a radiolabeled molecule or compound this may include radioactive tracers or radioactive prosthetic groups. The microfluidic device 10 is formed from a multi-layer construction and includes a sample layer 12 that includes a microfluidic channel 14 that is formed therein. The microfluidic channel 14 may include a serpentine shaped microfluidic channel as illustrated that has a number of turns back and forth across the sample layer 12. The microfluidic channel 14 includes ends 16, 18, one of which is used as an inlet for a fluid containing the radiolabeled molecule or compound while the other end is used as an outlet for the microfluidic channel 14. FIG. 1 illustrates a sample 100 contained in a sample reservoir 102 that contains a fluid or fluid mixture that includes a radiolabeled molecule or compound that is processed using the microfluidic device 10 that yields a concentrated 110 product.

In some embodiments, the microfluidic device or chip 10 may generate a more concentrated fluid that contains the radiolabeled molecule or compound. In other embodiments, a completely different fluid may be passed through the microfluidic device 10 to solvate a completely or partially dried radiolabeled molecule or compound. For example, the initial fluid that is loaded into the microfluidic device 10 may include an organic solvent while the final fluid that is ejected or retrieved from the microfluidic device 10 may include an aqueous-based fluid (e.g., water or saline). The sample layer 12 may include a number of holes or apertures 20 located around the edges or perimeter that are used to form the final device using one or more fasteners 56 as explained in further detail herein.

The microfluidic channel 14 may be formed in any number of ways that are typically used to form microfluidic channels. These include direct casting, laser ablation, CNC machining, injection molding, and the like. The sample layer 12 may be made from a number of different materials. Examples include, metals, and metal alloys (e.g., aluminum alloy), poly(methyl methacrylate) (PMMA), polytetrafluoroethylene (PTFE), glass-filled PTFE (glass-PTFE), polyetherimide, cyclic olefin copolymer (COC), polyether ether ketone (PEEK), polyamides (e.g., Nylon), polyamide-imide (PAI), poly-ether imide (PEI), polyphenylene sulfide (PPS), polybenzimidazole (PBI), thermoplastic polyimide (TPI), alloys of plastics, and glass. The dimensions of the microfluidic channel 14 may vary but generally has a width generally less than 4.5 mm (e.g., 2.25 mm) and a depth of around 0.05 mm with spacing of 0.5 mm between adjacent segments of the serpentine microfluidic channel 14.

The choice of material for the sample layer 12 may be selected based on the compatibility with the radiolabeled molecule or compound or the fluids in which the molecule or fluid is carried. In addition, certain materials, such as using an aluminum alloy, may produce higher evaporation rates. The thickness of the sample layer 12 may vary, particularly whether there is an optional compression layer 50 that is used as discussed herein. Typically, the thickness of the sample layer 12 may be several millimeters but may be thicker if the compression layer 50 is omitted.

Still referring to FIG. 1, the microfluidic device 10 includes a porous membrane 30 that is preferably formed of a hydrophobic polymer material. The porous membrane 30 includes pores 32 (seen in FIGS. 2A and 2B) therein that permit the passage of vapor from evaporating or gaseous fluid from the sample solution contained in the microfluidic channel 14. In one embodiment, the porous membrane 30 is made from PTFE and has a pore size of less than 0.5 µm. In particular, the pore size may be about 0.4 µm, 0.3 µm, 0.2 µm or less.

The microfluidic device 10 further includes a gas flow layer 40 that includes therein a gas-carrying channel 42 formed therein and corresponding in shape to the microfluidic channel 14. For example, the gas-carrying channel 42 may also include a serpentine shaped channel that matches that of the microfluidic channel 14 formed in the sample layer 12. In this regard, when the complete microfluidic device 10 is assembled, the gas-carrying channel 42 will mate with the microfluidic channel 14; being separated only by the porous membrane 30. In other embodiments, the gas-carrying channel 42 has a shape of configuration that does not match, exactly, the configuration of the microfluidic channel 14. For example, the porous membrane 30 may be permanently bonded to one or more of the layers 12, 40 in which case the exact matching is not necessary. The gas flow layer 40 is preferably formed from a metallic material such as a metal alloy such as aluminum. For example, in one embodiment, the gas flow layer 40 is made from 10 mm thick 6061 aluminum alloy block with a 3.5 mm deep gas-carrying channel 42 formed therein. The gas flow layer 40 serves, in one embodiment, as a conduit for heat to be applied to the fluid in the sample layer 12 as well as provides the gas-carrying channel 42 that is used to remove gas or vapor from evaporated fluid that passes through the pores 32 of the porous membrane 30. Holes may be provided in the gas flow layer 40 for the placement of cartridge heaters (not shown) which can be secured in place using thermal paste or the like. The gas flow layer 40 includes holes or apertures 44 along the edges or periphery that accommodate the fasteners 56. As an alternative to applying heat through the gas flow layer 40, a heating plate or block may be placed in contact with a surface of the microfluidic device 10 (including the sample layer 12).

In one embodiment, a compression layer 50 is also used to form the microfluidic device 10. The compression layer 50 is a thick (e.g., tens of mm thick) layer of rigid material that is applied to the surface (e.g., top surface) of the sample layer 12 and is used to provide structural support. The compression layer 50 may be made of an optically transparent material such as acrylic to allow for visual observation and monitoring if needed. The compression layer 50 includes holes or apertures 52 about the edges or periphery that are used to accommodate fasteners 56. The fasteners 56 may include bolts (and optional nuts), screws, clamps (even external clamps) or the like that are used to compress the various layers (i.e., sample layer 12, porous membrane 30, gas flow layer 40) together. Of course, in other embodiments, the compression layer 50 may be omitted entirely. In such instances, the sample layer 12 may need to be thicker to compensate for the lack of compression layer 50.

FIG. 2A illustrates a cross-sectional view of the fully assembled microfluidic device 10 taken along the line A-A of FIG. 1. FIG. 2A illustrates the direction of gas flow in the gas-carrying channel 42 along with the direction of heat that is generated in the gas flow layer 40. The sample fluid or solution is evaporated and passes through the pores 32 in the porous membrane 30 where the evaporated fluid can be removed from the microfluidic device using the gas flow. FIG. 2B illustrates another view of the fully assembled microfluidic device 10 taken along the line B-B of FIG. 1.

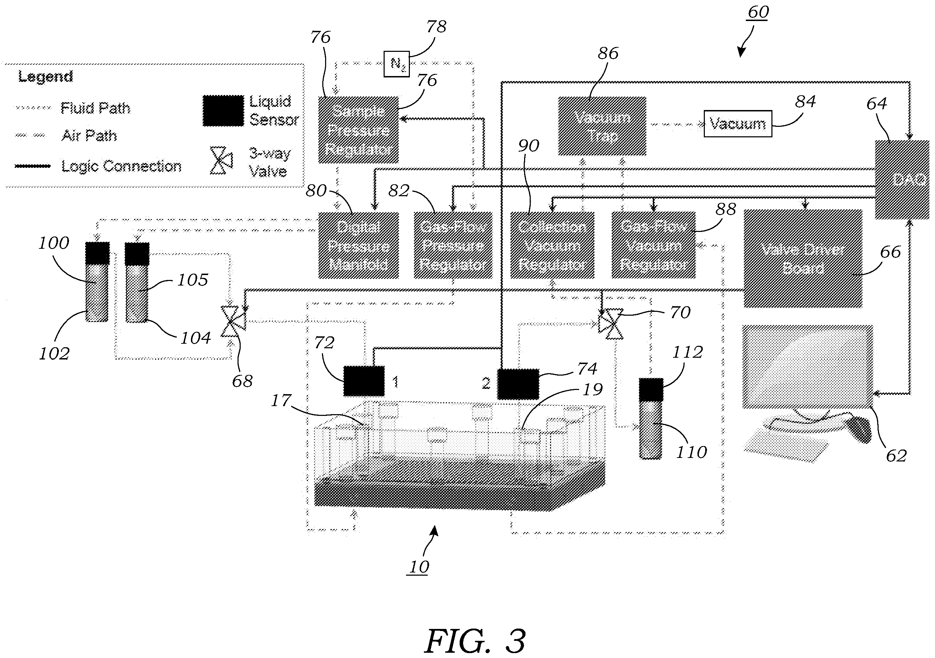

FIG. 3 illustrates an automated system 60 that incorporates the microfluidic device 10 described herein. The automated system 60 is able to automatically load a sample 100 contained in a sample reservoir 102 into the microfluidic device 10. Also illustrated is a rinse solution reservoir 104 that contains a rinse solution 105 (e.g., water, buffer, or the like) that can be input into the microfluidic device 10. An inlet 17 is located in the microfluidic device 10 which consists of a hole that is drilled or formed in the compression layer 50 and sample layer 12. Similarly, an outlet 19 is located in the microfluidic device 10 which consists of a hole that is drilled or formed in the compression layer 50 and sample layer 12. Tubing (not shown) is connected to the inlet 17 and the outlet 19 to provide fluid access to and from the microfluidic device 10. Still referring to FIG. 3, a computing device or computer 62 is provided that interfaces with various components of the automated system 60 via a data acquisition interface 64. The data acquisition module 64 interfaces with a valve driver board 66 that controls a three-way inlet valve 68. In another alternative embodiment, the valve drive board 66 may be omitted and the three-way inlet valve 68 may connect directly with the data acquisition module 64. The three-way inlet valve 68 is used to control flow of the sample 100 from the sample reservoir 102 as well as the rinse solution 105 from the rinse solution reservoir 104 to the microfluidic device 10. The valve driver board 66 is also used to control an outlet valve 70 that either blocks fluid from leaving the microfluidic channel 14 in the sample layer 12 or permits the fluid to leave the microfluidic device 10 and enter the concentrated product 110 in a collection reservoir 112.

The data acquisition module 64 interfaces with two liquid sensors 72, 74. A first liquid sensor 72 that is placed in the flexible tubing between the sample reservoir 102 and the inlet 17. A second liquid sensor 74 is positioned in the flexible tubing between the outlet 19 and the collection reservoir 112. The sensors 72, 74 are used to determine whether liquid or air are present in the tubing using voltages generated that are monitored by the data acquisition module 64. The data acquisition module 64 also is connected to a sample pressure regulator 76 that is used to adjust the pressure of a source of gas 78 (e.g., nitrogen). The source of gas 78 is used both to drive fluids (e.g., sample 100, rinse solution) through the microfluidic device 10 as well as used as the sweeping gas. Driving control of both the sample 100 and rinse solution 105 is accomplished using a digital pressure manifold 80 that connects to the data acquisition module 64. Pressure can selectively be applied to the sample reservoir 102 or the rinse solution reservoir 104 to push the solution into tubing connected to the three-way inlet valve 68. A gas flow pressure regulator 82 also connects to the data acquisition module 64 and is used to adjust the pressure of the gas (e.g., nitrogen) within the gas-carrying channel 42 of the gas flow layer 40. The system further includes a source of vacuum 84 (e.g., vacuum pump) that includes an in-line vacuum trap 86 that is connected a gas-flow vacuum regulator 88. The gas-flow vacuum regulator 88 interfaces with the data acquisition module 64 and is used to adjust the level of vacuum applied to the outlet or output of the gas-carrying channel 42. Similarly, a collection vacuum regulator 90 that also interfaces with the data acquisition module 64 is used to adjust the level of vacuum applied to the collection reservoir 112.

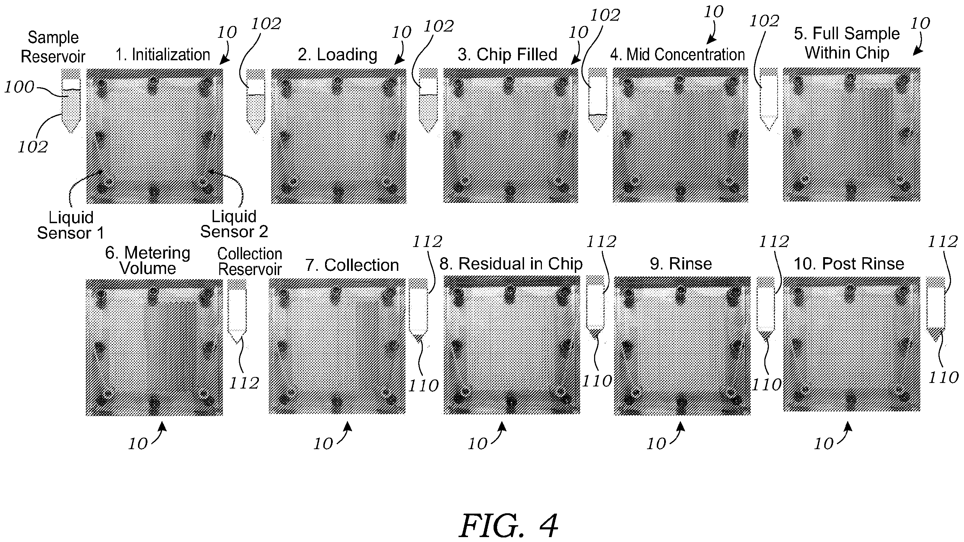

FIG. 4 illustrates a series of illustrative operational steps used to concentrate a sample 100 using the microfluidic device 10. Alongside each operational step is a photographic image of the microfluidic device 10. In this example, the sample 100 contains diluted food color dye which is used to visually observe the concentration process. The microfluidic device 10 first undergoes initialization and pre-heating as seen in initialization operation (1). Next, the sample reservoir 102 is pressurized to begin loading the sample as seen in loading operation (2). The microfluidic device 10 is then filled as seen in chip filled operation (3) and the sweeping gas is activated to begin the concentration process as seen in operation (4). When the sample volume has been reduced to the point where it just fits within the microfluidic device 10 (5), a timer is started and evaporation is continued to reduce the volume further as seen in (6). Next, the concentrated sample is then collected in operation (7) using collection reservoir 112, and any residual liquid in the microfluidic device as seen in (8) is recovered via rinsing in operation (9). Each rinse operation includes loading of rinse solution, partial evaporation to a set volume (i.e., same as operations (5, 6) above), and recovery of the concentrated rinse solution (9). After multiple rinsing steps (9), the microfluidic device 10 is free of residual sample (10).

In the example of FIG. 4, the fluid contained in the microfluidic device 10 was not fully evaporated. In other embodiments, the fluid contained in the microfluidic device 10 may be fully evaporated. For example, many radiolabeled molecules or compounds are typically synthesized in or contained in an organic solvent. Sometimes, the radiolabeled molecules or compounds are destined for injection in a mammal and the organic solvent component may be toxic; even at relatively low levels. In one embodiment of the invention, the fluid contained in the microfluidic device 10 is fully evaporated leaving a dried solute on the inner surface of the microfluidic channels 14 of the sample layer. The dried solute can then be re-dissolved in a different solvent that is run through the microfluidic device 10. For example, an aqueous-based fluid (e.g., water or saline) may be run through the microfluidic channels 14 after concentration (and drying has been performed) to recover the dried molecules or compounds. This new fluid which contains the dissolved or suspended radiolabeled molecules or compounds can then be ejected out of the microfluidic device 10 and collected. This collected product may, in some instances, be ready for direct injection. Alternatively, the collected product may undergo further processing for formulation and injection.

One particular benefit of the automated system 60 is that it rapidly enables the concentration of radioactive molecules or compounds (e.g., radiolabeled tracers) without the manual handling of radioactive materials. Evaporation rates in excess of 3 mL/min may be achieved. Further, the platform offers the ability to concentrate virtually any PET tracer because material selection for the sample layer 12 can be tailored to suit the particular compound of interest. Moreover, the smaller pores 32 in the porous membrane 30 significantly enlarge the range of compatible solvents that can be used with the microfluidic device 10. As explained herein, operating at 100°C operating temperature, the system 60 can handle samples with up to 80% (v/v) MeCN in H₂O, and up to 40% (v/v) EtOH in H₂O without significant loss of the sample. While these limits include the majority of mobile phases used in the production of PET tracers, these limits of organic solvent content could likely be further extended if needed, by decreasing operating temperatures (at the expense of evaporation rate) or by using a porous membrane 30 with even smaller pore dimensions.

### Experimental

### Microfluidic concentrator design and fabrication

The microfluidic device 10 was designed to perform sweeping-gas membrane distillation, in which a fluid sample and a sweeping gas are separated by a hydrophobic, polymer porous membrane 30. The polymer porous membrane 30 prevents passage of the liquid as long as the contact angle is > 90°, but vapor generated as the aqueous sample is heated can pass through the polymer porous membrane 30 and into the sweeping gas stream. In distillation, the vapor carried away is condensed and collected for downstream use; however, in the case of sample concentration, the vapor is discarded, and the reduced volume of sample (containing the desired solute in more concentrated form) is collected of-chip.

The microfluidic device 10 measured 120 mm x 120 mm in lateral dimensions. The compression layer 50 (25.4 mm thick acrylic) provides optical transparency for visual monitoring of the concentration process and rigidity for clamping all layers together. The sample layer 12 is made out of 2 mm thick plastic with a patterned serpentine microfluidic channel 14 (2.25 mm wide x 0.05 mm deep channels with 0.5 mm spacing). The microfluidic channel 14 has rounded corners or turns to avoid dead volumes that interfere with efficient sample recovery, and was designed with a small channel width (2.25 mm) to avoid "sagging" of the porous membrane 30 while still maintaining a large evaporation surface area (∼57 cm²). The open side of the sample microfluidic channel 14 is in contact with a porous membrane 30 (PTFE, 0.2 µm pore size; Sterlitech, Kent, WA, USA). The gas flow layer 40 is a 10 mm thick 6061 aluminum alloy block with a 3.5 mm deep serpentine microfluidic channel 14 matching the sample layer (CNC machined by Proto Labs, Inc., Maple Plain, MN, USA). This layer served to provide heat, and the channel carried the sweeping gas. The layers are clamped together using bolts 56; small deformation of the membrane layer 30 ensures good sealing along channel walls to both the sample layer 12 and gas flow layer 40.

The sample layers 12 were formed from a variety of materials, including: poly(methyl methacrylate) (PMMA), polytetrafluoroethylene (PTFE), glass-filled PTFE (glass-PTFE), polyetherimide (Ultem™), cyclic olefin copolymer (COC) and polyether ether ketone (PEEK), and aluminum alloy. The plastics were chosen for their chemical inertness, high temperature stability, and/or transparency. An exemplary PMMA sample layer 12 was fabricated by Aline Inc. (Rancho Dominguez, CA, USA) via laser ablation, and then sample layers of all materials were fabricated via CNC machining by Delmar Company (Lakeville, MN, USA). Holes or apertures 52, 20 through the acrylic top layer 50 and sample layer 12 provide fluid access to the inlet and outlet of the sample channel. Similarly, holes 44 through the gas flow layer 40 provide access to the inlet and outlet of the sweeping gas channel 42. To distinguish the two methods of fabricating PMMA layers, they are referred to as PMMA-machined and PMMA-ablated herein.

### Automated concentrator system

To automate its operation, the microfluidic device 10 was integrated into the system 60 shown in FIG. 2. A reservoir 102 for the sample 100 and a reservoir 104 for the rinsing solution 105 (e.g. saline, to help collect the sample after concentration) include 15mL conical tubes (352096, BD Biosciences, San Jose, CA, USA). Two holes were drilled in the cap of each tube, one for 1/8" polyurethane tubing to pressurize the reservoir, and one for 1/16" OD ETFE tubing (1517L, IDEX Health & Science, Oak Harbor, WA, USA) to deliver the reservoir contents. Tubing was sealed in place with a hot glue gun. The fluid delivery lines from the reservoirs 102, 104 are connected to the two inlet ports of an electronic 3-way valve 68 (LVM105R, SMC Corporation, Japan). The output of this "sample inlet valve" 68 is connected via 1/16" OD ETFE tubing to the sample inlet of the concentrator microfluidic device or chip 10. An electronic pressure regulator 82 (ITV0010-2BL, SMC) connected to a nitrogen source 78 and supplies pressure to the reservoirs through independently controlled solenoid valves (S070B-5DG, SMC).

The sample outlet from the microfluidic device 10 is connected via 1/16" OD ETFE tubing to another 3-way valve 70 to choose whether the outlet is blocked (during evaporation) or connected to the sample collection reservoir 112. The collection reservoir 112 (i.e., vial) is connected via a vacuum trap 86 to a digitally controlled vacuum regulator 88 (ITV2090, SMC) which in turn was connected to a central "house" vacuum source 84. This vacuum source 84 was capable of pressure as low as -90 kPa with a vacuum capacity of 1.9 L/min. The gas-carrying channel 42 of the gas flow layer 40 was connected via 1/4" polyurethane tubing through an electronic pressure regulator 82 (ITV2010, SMC) to the nitrogen source 78, and through a vacuum regulator 88 (ITV2090, SMC) to the vacuum pump.

The regulators and valves were digitally controlled using a data acquisition module 64 (NI USB-6009, National Instruments, Austin, TX, USA). A custom-built Darlington transistor array board was used to step up the voltage and current needed to drive the 3-way valves 68, 70. Two tubing fluid sensors 72, 74 (OCB350L062Z, Optek Technologies, Carrollton, Texas, USA) are positioned on the 1/16" tubing between the sample inlet valve 68 and the concentration inlet to the microfluidic device 10 (sensor 72) and on the 1/16" tubing between the concentrator microfluidic device 10 outlet and the sample collection valve 70 (sensor 74). Analog voltage readings from the sensors 72, 74 are connected to the data acquisition module 64 (NI USB-6009, National Instruments) for computer analysis using the computer 62. Comparison to a threshold value is used to determine if the tubing under the sensor 72, 74 is filled with air or liquid.

Heat was provided to the microfluidic device 10 by 100W cartridge heaters (8376T27, McMaster Carr, Santa Fe Springs, CA, USA) inserted into holes drilled in the metal gas flow layer. Thermal paste (OT-201-2, OMEGA Engineering, Inc., Stamford, CT, USA) was used to provide good thermal contact. For each cartridge heater, a K-type thermocouple (5TC-GG-(K)-30-(72), OMEGA Engineering, Inc., Stamford, CT, USA) was also inserted into the heating block near the heater. Feedback control of each heater-thermocouple pair was performed with an independent PID temperature controller (CN7500, OMEGA Engineering, Inc., Stamford, CT, USA). Control parameters were set by the "auto-tune" feature.

The heating power was selected based on the following considerations. To heat water from room temperature to 100°C and convert it to vapor form at a rate of ∼3 mL/min requires ∼130W of power. Empirical testing showed that the theoretical minimum power was not sufficient to maintain the temperature of the heating block, presumably due to other thermal losses and the finite time needed for transport of heat from the cartridge heaters to the sample channel. Using two 100W heaters during 100°C evaporation, the measured temperature was observed to be ∼6°C below the set-point. On the other hand, with four 100 W heaters, the measured temperature matched the set-point within ±1.5°C, and this configuration was used for all experiments. Further increased heating power (e.g., four 300W heaters) could heat the chip 10 more quickly but gave similar temperature stability and was not used because the chip could be pre-heated prior to use.

### Concentrator operation

The concentration process (illustrated in FIG. 4) was automated using a custom-written LabVIEW (National Instruments) program based on a finite state machine architecture and executed using computer 62. Prior to the concentration process, the microfluidic device 10 is first heated to the desired temperature (∼5 min to reach steady state), the sample collection valve 70 is closed, and the sample inlet valve 68 is switched to the "sample" position. Sample is loaded into the chip 10 by pressurizing the sample vial, to a pressure (*Pₛₐₘₚₗₑ*) that is constantly applied for the duration of the concentration process. Air initially in the system ahead of the sample readily escapes by passing through the porous membrane 30 into the gas-carrying channel 42 of the gas flow layer 40, allowing the sample to reach the chip 10. The sample advances until the microfluidic channel 14 is filled and sample begins to emerge from the outlet of the chip 10 (due to compression of the remaining trapped air), triggering sensor 74. At this point, the sweeping gas flow is initiated (by applying *P_{gas_in}* and *P_{gas_out}* at the gas flow inlet and outlet, respectively). As solvent evaporates, additional space is created within the microfluidic channel 14, allowing new sample to enter the microfluidic device 10. The solute becomes progressively more concentrated within the microfluidic device 10.

When the sample reservoir 102 is exhausted, the trailing end of the sample passes through sensor 72 (i.e. liquid to air transition), and the concentrated sample volume matches the chip volume. The sample volume can be further reduced by continuing the heating process for an additional delay time to achieve a final volume smaller than the chip volume. This delay is needed because even though the designed chip volume is ∼0.29 mL, the collected volume without further evaporation is ∼2.75 mL. This discrepancy is presumably due to significant deflection of the porous membrane 30 under the operating conditions. The actual delay time was varied, depending on the mode of operation. When performing partial solvent evaporation, a delay time of 50 s was used, resulting in a recovered volume from the chip of 0.4-0.5 mL. On the other hand, when performing complete solvent evaporation, the delay time was typically set to 270 s (corresponding to ∼220 s for water to completely evaporate at 100°C plus a safety margin.)

When operating in partial evaporation mode, the concentrated sample is first collected, and then the microfluidic device 10 is rinsed multiple times. In complete evaporation mode, there is no initial collection step and rinsing is performed directly after complete dryness is achieved. To collect the concentrated sample, the sample collection valve 70 opens after the delay time, and the sample is driven to the collection reservoir 112 by the sample inlet pressure. During the collection process, the vacuum connected to the collection reservoir 112 (P_{vacuum}) is turned on at -1.0 psi. The sample is collected for a set period of time that is empirically determined; for the concentrated sample volume resulting from 50s delay time, ∼4s was sufficient for collection, but 14s was chosen to incorporate a safety margin. Next, the vacuum is ramped to - 8.8 psi (P_{Vacuum_ramp}) to recover any residual fluid trapped within the fluidic path of the microfluidic channel 14. Residual fluid recovery is typically complete within ∼3s, but 9s was chosen to include a safety margin. Gradually ramping up the vacuum pressure was needed to prevent the fluid from breaking up in the tubing.

To perform a rinse, the sample collection valve 70 is closed, and the sample inlet valve 68 is switched to the rinsing solution reservoir 104. The rinse solution reservoir 104 is pressurized to *Pₛₐₘₚₗₑ,* and rinse solution 105 is loaded just as initially performed for the sample 100. However, when it reaches sensor 74, the sample inlet valve 68 is switched back to the empty sample reservoir 102 (which is still pressurized at *Pₛₐₘₚₗₑ*)*.* At this point, the total volume of rinse solution (including that in the chip, tubing, etc.) is ∼3 mL. The rinse solution 105 is concentrated just like the initial sample, and when the trailing end of the rinse solution 105 passes sensor 72, a timer is started for additional delay time. Finally, the concentrated rinse plug is ejected in the same fashion as the original sample. Multiple rinse steps can be performed to improve sample recovery.

### Measuring Evaporation Rate

Evaporation rate was measured by placing a 5 mL Fisherbrand graduated serological pipet (13-678-11D, Fisher Scientific, Pittsburgh, PA, USA) inline between the sample reservoir 102 and the chip 10. Connections were made via 1/4" OD polyurethane tubing (TIUB07, SMC Pneumatics, Yorba Linda, CA). Evaporation rates were measured by observing the sample fluid meniscus as it passed through the graduated pipette. The amount of time it took for the fluid meniscus to move by 1 mL was recorded and used to calculate the evaporation rate. Evaporation rates were determined for five successive 1 mL increments and averaged.

For some experiments, the evaporation rate was examined over a longer period of time (larger volume evaporated). For these cases, the sample reservoir 102 was replaced with a larger conical tube (50mL Falcon conical tube; Corning Inc., Corning, NY, USA), and a larger graduated pipette (50 mL Fisherbrand serological pipet, Fisher Scientific, Pittsburg, PA, USA) was used to monitor liquid movement. Though vertical orientation of the pipette adds a hydrostatic pressure to the sample pressure (∼0.4 psi in the worst case) this pressure was found to have negligible effect on evaporation rate.

### Reagents

Ethanol (EtOH; 200 proof) was purchased from the UCLA Chemistry Department (Los Angeles, CA, USA). Anhydrous acetonitrile (MeCN), ammonium dihydrogen phosphate (NH₄H₂PO₄), potassium carbonate (K₂CO₃), dimethyl sulfoxide (DMSO), and trifuoroacetic acid (TFA) were purchased from Sigma-Aldrich (Milwaukee, WI USA). Deionized water was obtained from a Milli-Q water purification system (EMD Millipore Corporation, Berlin, Germany). Food dye used in the experiments was purchased from Kroger (Cincinnati, OH, USA). Food dye was diluted with 18MΩ deionized water in the ratio of 1:50 v/v. Saline (0.9% w/v) was purchased from Hospira (Lake Forest, IL, USA). [¹⁸F]fluoride in [¹⁸O]H₂O was obtained from the UCLA Biomedical Cyclotron (Los Angeles, CA, USA). 4,7,13,16,21,24-hexaoxa-1,10-diazabicyclo[8.8.8]hexacosane (Kryptofix 222; K222) was purchased from ABX (Radeberg, Germany). Unless otherwise noted, all materials were used as received.

1-(2'-deoxy-2'-[¹⁸F]-β-D-fluoroarabinofuranosyl) cytosine ([¹⁸F]D-FAC), 2-deoxy-2-[¹⁸F]fluoro-5-ethyl-β-D-arabinofuranosyluracil([¹⁸F]D-FEAU), (S)-N-((1-allyl-2-pyrrrolidinyl)methyl)-5-(3-[¹⁸F]fluoropropyl)-2,3-dimethoxybenzamide ([¹⁸F]fallypride), and 9-(4-[¹⁸F]fluoro-3-hydroxymethylbutyl)-guanine ([¹⁸F]FHBG) were synthesized using the ELIXYS radiosynthesizer (Sofie Biosciences, Culver City, CA, USA). Synthesis of N-[2-(4-[¹⁸F]fluorobenzamido)ethyl]maleimide ([¹⁸F]FBEM) was synthesized on the ELIXYS radiosynthesizer by straightforward adaptation of literature methods. All tracers and prosthetic groups were purified using semi-preparative HPLC, and the collected fraction of each (suspended in its respective mobile phase; see Table 1 below) was used directly without further formulation. [¹⁸F]D-FAC was formulated for use in imaging as described below.

**Table 1**

| Tracer | HPLC Mobile Phase (all ratios are v:v) | Flow Rate (mL/min) | Purification Method |
|---|---|---|---|
| [¹⁸F]D-FAC | 1:99 EtOH/10mM NH₄H₂PO₄ | 5.0 | Isocratic |
| [¹⁸F]FHBG | 5:95 MeCN/50mM NH₄OAc | 5.0 | Isocratic |
| [¹⁸F]D-FEAU | 8:92 MeCN/water | 5.0 | Isocratic |
| [¹⁸F]FBEM | 20:80 MeCN/water | 5.0 | Isocratic |
| [¹⁸F]Fallypride | 60:40 MeCN/25mM NH₄HCO₂ with 1%TEA | 5.0 | Isocratic |

### Determining Operating Conditions

Briefly, *Pₛₐₘₚₗₑ* should be high enough that the chip 10 remains full of sample during evaporation. In addition, the highest pressure in the gas-carrying channel 42 must be less than the sample pressure, i.e. P_{gas_in} < *Pₛₐₘₚₗₑ.* Finally, the largest pressure difference across the porous membrane 30 must not exceed the breakthrough pressure (BTP), i.e. *Pₛₐₘₚₗₑ - P_{gas_out}* < BTP. BTP is defined as the applied pressure at which sample (in liquid form) can directly leak through the pores 32 of the porous membrane 30 into the gas-carrying channel 42. It is closely related to the concept of capillary pressure, Pc, which is the pressure needed to cause fluid to enter a pore with radius *r,* BTP ≈ *Pc* = 2γcos(π-θ)/*r*, where γ is the surface tension of the sample (at an air interface) and θ is the contact angle of the liquid sample with respect to the porous membrane 30 surface. Since γ and θ depend on the composition of the mobile phase and temperature, the BTP is a function of these variables as well. Spontaneous permeation of the membrane (i.e. 'breakthrough') always occurs when θ < 90°. For θ > 90°, permeation of the membrane only occurs when the fluid sample is pressurized against the membrane with a pressure exceeding the BTP. First, measurements of contact angle for various solvent compositions and temperatures were made to determine the conditions under which θ > 90°, indicating the extent of the operating range in terms of solvent composition and temperature. Since it is important to operate under an adequate safety margin, more direct measurements of BTP were also determined for various solvent compositions and temperatures. Once BTP was known, other operating parameters were selected to meet the above constraints.

### Determining Sample Recovery Efficiency

The efficiency of sample recovery (i.e., amount of solute recovered compared to amount in initial sample) was quantitatively evaluated using radioactive solutions. Experiments used either a [¹⁸F]fluoride solution or a solution of an ¹⁸F-lableled PET tracer. [¹⁸F]fluoride solutions consisted of 0.3-1.25 mCi of [¹⁸F]fluoride, 2.25 mg K222 (0.6 mM final concentration) and 0.41 mg of K₂CO₃ (0.3 mM final concentration) in 10 mL ddH₂O. The amount of solute directly corresponds to the amount of radioactivity, which was measured using a calibrated dose calibrator (CRC-25 PET, Capintec Inc., Ramsey, NJ). Labeled PET tracers were suspended in their respective HPLC mobile phase (Table 1) and had activity levels ranging from 0.2-1.0 mCi.

Radioactivity measurements were made of the original sample, the collected concentrated sample and of each five subsequent rinse steps. In the case of complete solvent evaporation method, the collected sample is obtained from an initial rinse step. Measurements were corrected for radioactive decay, and the fraction of initial radioactivity was calculated for each portion of the output volume. Tracer stability at operating temperatures was assessed via analytical radio-HPLC. After partial or complete evaporation methods, the organic solvent content of several samples was assessed via gas chromatography mass spectrometry (GC-MS).

### Results and Discussion

### Scope of HPLC mobile phases

In the synthesis of PET tracers and radiolabeled prosthetic groups (for labeling biological molecules), HPLC purification methods nearly always use mixtures of EtOH in water or MeCN in water, sometimes with additives to buffer the pH to improve separation or stability. Examples of mobile phases for purification of several radiolabeled molecules are listed in Table 1.

Contact angles were examined in order to determine the range of solvent compositions and temperatures for which θ > 90° (i.e. for which no spontaneous breakthrough would be expected). For mobile phases consisting of MeCN and water mixtures, it was observed that θ > 90° for all compositions (from 0 to 100% MeCN in water, v/v) and all temperatures (RT to 80°C). For EtOH, concentrations up to 70% EtOH in water (v/v) can be sustained (θ > 90°) at all temperatures (RT to 80°C) without breakthrough, but compositions ≥ 80% EtOH (v/v) would not be suitable at any temperature. Unfortunately, for temperatures above 80 °C, droplets rapidly evaporated, preventing accurate contact angle measurement, and thus this method does not provide a good way to estimate the behavior under the most aggressive temperatures. For both solvents, the effect of composition on wetting property appears to be more significant than the effect of temperature. This is expected because Eötvös rule describes a linear decrease in surface tension with increasing temperature, but surface tension has been observed to decrease superlinearly with increasing mole fraction of organic solvent.

While the contact angle gives an idea about spontaneous wetting, it does not indicate whether the BTP is sufficiently large for proper operation of the chip without breakthrough in practice. Using a specially designed test rig, measurements of BTP for various solvent compositions and temperatures (up to 80°C) were made (see Tables 2 and 3 below; BTP is measured in psi; N.M. indicates not measured).

**Table 2**

| % MeCN in H₂O (v/v) | Temperature (°C) | | | | |
|---|---|---|---|---|---|
| | RT | 40 | 60 | 80 | 100 |
| 0 | > 13.5 | > 13.5 | > 13.5 | > 13.5 | N.M. |
| 10 | > 13.5 | > 13.5 | > 13.5 | > 13.5 | N.M. |
| 20 | > 13.5 | > 13.5 | > 13.5 | > 13.5 | N.M. |
| 30 | > 13.5 | > 13.5 | > 13.5 | > 13.5 | N.M. |
| 40 | > 13.5 | > 13.5 | > 13.5 | > 13.5 | N.M. |
| 50 | > 13.5 | > 13.5 | > 13.5 | > 13.5 | N.M. |
| 60 | > 13.5 | > 13.5 | > 13.5 | > 13.5 | N.M. |
| 70 | > 13.5 | > 13.5 | > 13.5 | > 13.5 | N.M. |
| 80 | > 13.5 | 12.2 ± 0.5 | 11.6 ± 1.4 | > 13.5 | N.M. |
| 90 | 12.0 ± 0.2 | 10.6 ± 0.8 | 8.6 ± 0.5 | 8.1 ± 0.3 | N.M. |
| 100 | 7.3 ± 0.3 | 5.6 ± 0.2 | 3.3 ± 0.4 | 2.0 ± 0.4 | N.M. |

**Table 3**

| % EtOH in H₂O (v/v) | Temperature (°C) | | | | |
|---|---|---|---|---|---|
| | RT | 40 | 60 | 80 | 100 |
| 0 | > 13.5 | > 13.5 | > 13.5 | > 13.5 | N.M. |
| 10 | > 13.5 | > 13.5 | > 13.5 | > 13.5 | N.M. |
| 20 | > 13.5 | > 13.5 | > 13.5 | > 13.5 | N.M. |
| 30 | > 13.5 | > 13.5 | > 13.5 | > 13.5 | N.M. |
| 40 | > 13.5 | > 13.5 | > 13.5 | > 13.5 | N.M. |
| 50 | 11.5 ± 1.2 | 11.3 ± 0.7 | 12.0 ± 0.2 | > 13.5 | N.M. |
| 60 | 7.9 ± 0.3 | 7.6 ± 0.3 | 7.6 ± 0.2 | 12.7 ± 0.5 | N.M. |
| 70 | 4.4 ± 0.2 | 4.1 ± 0.2 | 4.2 ± 0.3 | 5.4 ± 0.6 | N.M. |
| 80 | 1.8 ± 1.0 | 0.4 ± 0.4 | < 0.5 | 2.2 ± 0.8 | N.M. |
| 90 | < 0.5 | < 0.5 | < 0.5 | 3.9 ± 0.3 | N.M. |
| 100 | < 0.5 | < 0.5 | < 0.5 | 5.5 ± 0.2 | N.M. |

As expected, for samples containing either solvent (MeCN and EtOH), an increase in temperature or organic solvent composition results in decreased breakthrough pressure. For MeCN/water mixtures, BTP was very high (> 13.5 psi) for all compositions up to 70% MeCN v/v (at all temperatures), and was moderately high (> 7.5 psi) for all compositions up to 90% MeCN v/v (at all temperatures). Moderate BTP values still allow considerable freedom in choice of operating conditions (*Pₛₐₘₚₗₑ, P_{gas_in},* and *P_{gas_out}*)*.* Only at 100% MeCN at elevated temperatures is the BTP low enough that reliable operation may be difficult. For EtOH/water mixtures, on the other hand, BTP was very high (> 13.5 psi) for compositions up to 40% EtOH v/v at all temperatures tested, and was moderately high (>7.5 psi) for compositions up to 60% EtOH v/v at all temperatures. For all compositions ≥70% EtOH v/v, the BTP is low enough that reliable and efficient operation may be difficult. These results correspond well to the contact angle data.

### Operating conditions

A lower limit of BTP of ∼7.5 psi was chosen as a good compromise of compatibility with a wide range of solvent mixtures and temperatures as well as ensuring some room for optimizing the operating conditions. As a starting point, the following parameters were selected *Pₛₐₘₚₗₑ* = 5.3 psi, P_{gas_in} = 4.5 psi, and *P_{gas_out}* = -0.25 psi. *Pₛₐₘₚₗₑ* was sufficient to ensure the chip 10 was always filled with sample under all conditions. The maximum pressure across the membrane is 5.6 psi, which is below 7.5 psi by a 35% safety margin. P_{gas_in} is below *Pₛₐₘₚₗₑ* by a ∼20% safety margin. A limited exploration of gas-flow pressure differential and sample pressure was performed to determine the effect on evaporation rate performance. Over the range tested, *Pₛₐₘₚₗₑ* was found to have negligible effect on the evaporation rate. The gas-flow pressure differential did have a small effect. Changing to a differential of 6.0 psi instead of 4.75 psi would increase evaporation rate by ∼ 5%, but if similar safety margins were used, the minimum BTP that could be tolerated would be -10 psi, which would exclude several solvent compositions; thus this change was not implemented. Parameters related to sample collection were set to maximize sample recovery. *P_{vacuum}* ≤ -2.0 psi resulted in break-up of the sample in tubing during recovery, so *P_{vacuum}* was set to -1.0 psi. This was ramped up to *P_{vacuum_ramp}* = -8.8 psi, near the best vacuum that could be achieved with the pump used.

### Evaporation rate

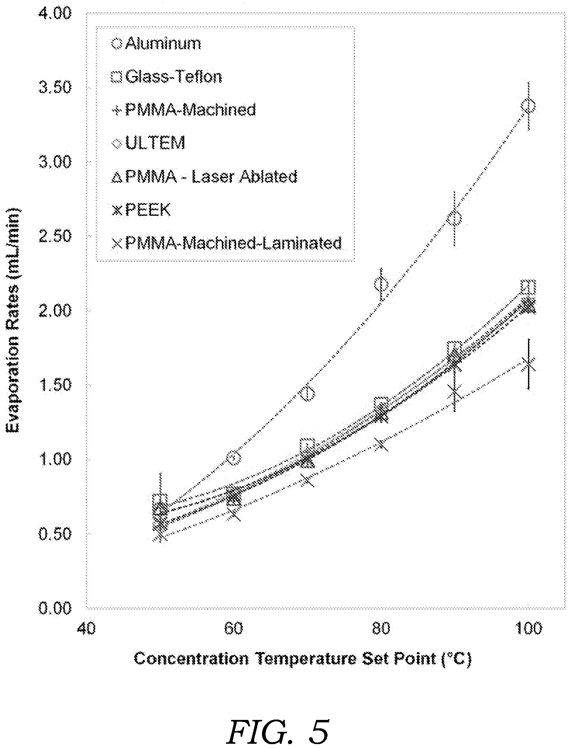

Using the above operating conditions, evaporation rates were extensively characterized. Maximum evaporation rates of water at 100°C exceeded 2 mL/min. Concentration was also performed using a rotary evaporator according to parameters recommended by the manufacturer. It was found that the evaporation rate at 80°C to be 1.03 ± 0.04 mL/min compared with 1.53 ± 0.02 mL/min achieved in the microfluidic chip 10 at the same temperature. The observed evaporation rate of the chip 10 depended somewhat on the material that was used for the sample layer as seen in FIG. 5. Evaporation rates using PMMA-machined, PMMA-ablated, polyetherimide (Ultem™), and PEEK, were very similar across the temperature set-points. The evaporation rate for glass-Teflon is significantly higher than the other materials. The differences may be related to the thermal conductivity of the sample layer materials. Thermal conductivities of PMMA, polyetherimide (Ultem™), and PEEK are all similar (0.19, 0.22, 0.25 W/mK, respectively), whereas the thermal conductivity of glass-PTFE is significantly higher (0.42 W/mK).

In addition to comparing sample layer materials, different chip architectures were explored to compare the effect of heater placement, including sample layer heating wherein heat is applied by a heating block in direct thermal contact with the sample layer. Most designs had similar or inferior performance with the exception of one design having a 3.2 mm thick 6061 aluminum alloy sample layer 12 (thermal conductivity 167 W/m^{∗}K) instead of a plastic sample layer 12. In this configuration both the sample layer 12 and the gas flow layer 40 were made from metal. The aluminum alloy sample layer 12 did not include microfluidic channels 14 formed therein. Instead, the microfluidic channels 14 were formed due to the deflection of the porous membrane 30 into the gas-carrying channel 42; making room for sample fluid. At 100°C, evaporation rate was 3.38±0.16 (n=5) mL/min compared to 2.02 ± 0.04 mL/min (n=5) for the PEEK sample layer, though the results are not directly comparable due to different arrangement of heaters. Because the opaqueness of the metal prevents convenient visualization, the plastic sample layers were used for all remaining experiments, but this result shows the potential improvement that may be possible by considering materials with high thermal conductivity.

### Effect of solutes and solvents on evaporation rate

Radiolabeled molecules may be in HPLC mobile phase volumes as large as ∼50 mL prior to concentration and formulation using the microfluidic device 10, leading to large increases (∼50x) in concentration of salts and additives that may be present in the mobile phase. Though an increase in solute concentration can lower the vapor pressure (which would be expected to reduce the evaporation rate), it was found that evaporation rates of 50 mL samples were remarkably constant throughout the entire evaporation process. Solutes became a factor during concentration only when the starting concentration of salts was 10x higher than normally used in HPLC mobile phases.

The effect of organic solvents was also considered. Not surprisingly, increasing concentration of organic solvent in the mobile phase led to increase in evaporation rate. For large sample volumes, it was expected that the evaporation rates to be initially higher and then decrease as the lower boiling point organic solvent was evaporated off leaving mostly water within the chip. However, it was found experimentally that the evaporation rates remained nearly constant.

### Sample Recovery

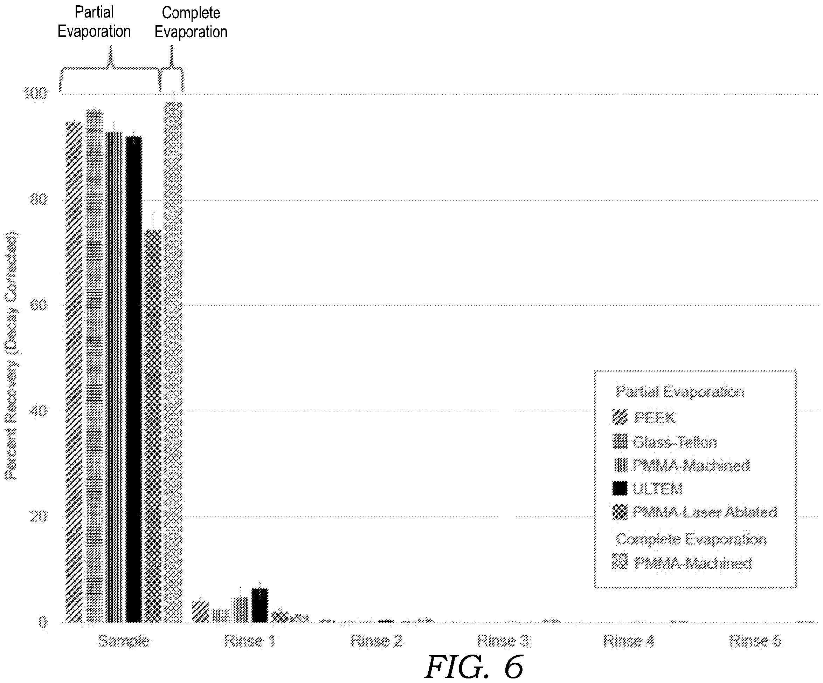

After recovery of the concentrated sample, additional rinse steps are used to recover residual amounts of the sample. However, because rinse steps have the undesirable effect of increasing the final collected volume, the effect of rinsing was experimentally examined so the number of rinse steps could be optimized. Using samples containing [¹⁸F]fluoride, the amount of radioactivity recovered in the initially ejected plug and five subsequent rinse plugs is plotted in FIG. 6. For the majority of materials used as sample layers (glass-PTFE, polyetherimide (Ultem™), PEEK, PMMA-machined), 92-97% of [¹⁸F]fluoride is recovered from the initial ejection. Following the initial ejection with two (2) sample rinses increases [¹⁸F]fluoride recovery for all materials (>97%). Subsequent rinses recover negligible amounts of activity.

On the other hand, using PMMA-ablated, recovery following two rinses was poor (74 ± 3%, n=3). Since PMMA fabricated by CNC machining exhibited excellent recovery, this result is most likely explained by either higher surface roughness of the laser ablated surface, or chemical modifications taking place during laser ablation. Laser ablated PMMA was therefore not used in further experiments. In practice, it is likely that sample layers would be manufactured via injection molding and should be immune to this issue. Sample recovery performance was also tested for cases where the system was operated in complete evaporation mode (FIG. 6). An experiment using PMMA-machined sample layer showed 98 ± 2% (n=3) recovery with the initial ejection and 100 ± 2% (n=3) recovery with two additional rinses.

### In-chip measurements of breakthrough

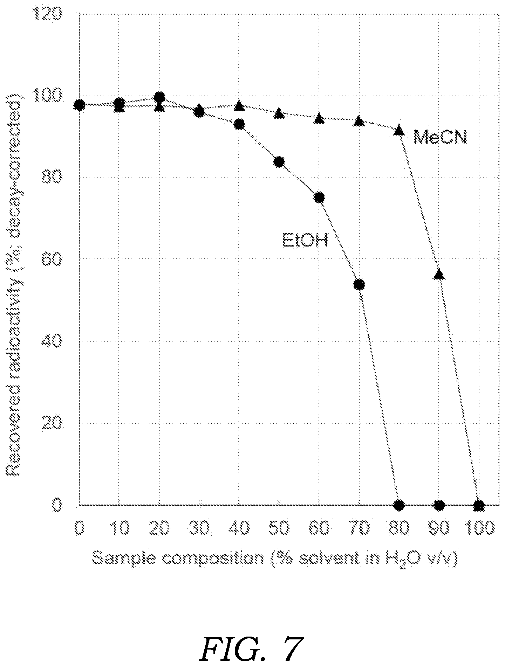

Sample recovery experiments also provided a way to measure BTP *in situ.* Measurements of contact angle or BTP using the test rig had limitations at high temperatures and could not make measurements at all >80°C. Loss due to breakthrough was monitored using 10 mL samples of different solvent compositions containing 0.6mM K_{2.2.2}. and 0.3mM K₂HCO₃ spiked with ∼5-10 µL of a solution containing [¹⁸F]fluoride (-0.5 mCi). Results are summarized in the FIG. 7. It was found that concentrations of MeCN up to 40% can be concentrated at 100°C with almost no sample loss, and only minor loss (<6%) when MeCN content is 40-80%. EtOH compositions up to 20% showed no apparent activity loss, and solutions with 30-40% EtOH showed minor (<7%) loss. Interestingly, the loss is not a binary effect, i.e. zero loss below breakthrough and 100% loss above breakthrough. This may be explained by slight non-uniformity in the porous membrane 30 (e.g., where some regions have slightly larger pore size, with lower BTP), or by the pressure gradients in the system (i.e., such that BTP is exceeded only near the sample outlet, where the pressure across the membrane is greatest). In either case, breakthrough may only occur over a small fraction of the porous membrane 30 area, limiting its effect. Since losses of 6-7% would be considered acceptable, one can conclude that mobile phases with up to 80% MeCN in water (v/v), or up to 40% EtOH in water (v/v) can be concentrated with the microfluidic device 10 at all temperatures up to 100°C. This range is sufficient to cover all HPLC mobile phases used in the purification of PET tracers.

### Concentration of PET tracers

### Partial evaporation method

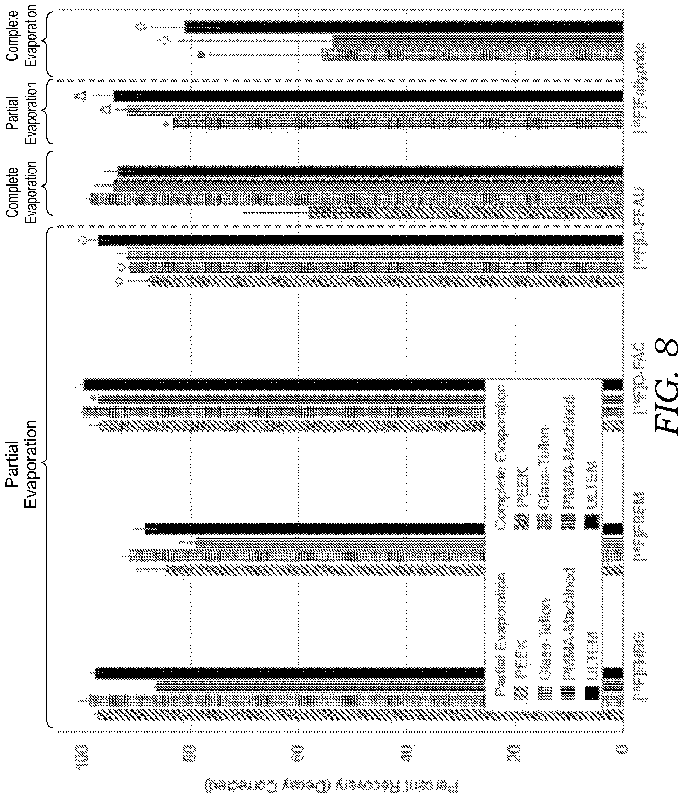

As a demonstration, batches of four PET tracers ([¹⁸F]D-FAC, [¹⁸F]FHBG, [¹⁸F]Fallypride and [¹⁸F]FEAU) as well as a prosthetic group for peptide and protein labeling ([¹⁸F]FBEM) were concentrated with the system. Initially, the partial evaporation method (100°C, plus 2 rinse steps) was explored. Results are summarized in FIG. 8. [¹⁸F]D-FAC was concentrated with recovery >96% for all chip materials. Concentration of [¹⁸F]FHBG using PEEK, polyetherimide (Ultem™) and glass-Teflon exhibited >97% recovery, but recovery was significantly lower for PMMA-machined, i.e. 86.2 ± 0.4% (n=3). Recovery of concentrated [¹⁸F]Fallypride was 94.2 ± 4.6% (n=4) with the Ultem™ chip, slightly lower (91.7± 2.2%, n=4) for the PMMA-machined chip, and only 83.1% (n=1) for the glass-Teflon chip. (PEEK was not tested.) For [¹⁸F]FEAU, recovery in the Ultem™ chip was 97.1 ± 1.9% (n=2). Slightly lower recovery was seen in the PMMA-machined chip (91.1 ± 0.2%, n=3) and glass-Teflon chips (91.8 ± 1.7%, n =2), and significantly worse performance was observed for PEEK (87.8 ± 4.0%, n=2). After concentration, the prosthetic group [¹⁸F]FBEM was recovered with 91.2 ± 1.2% (n=3) recovery in the glass-PTFE chip. The PEEK and Ultem™ materials produced slightly lower recovery rates of 84.5 ± 5.3% and 88.4 ± 2.0%, respectively, and PMMA-machined performed the worst (recovery of 79.0 ± 2.9%, n=3). Partial evaporation could be completed at a rate of ∼2 mL/min (depending on conditions) plus a 4.0 min "overhead" for sample loading and final rinse steps.

In post-experiment analysis, residual radioactivity was found to be localized on the sample layer (rather than on the Teflon porous membrane 30) suggesting adverse tracer interaction is with the sample layer surface. Considering that the layers were fabricated using the same milling method resulting in similar surface finish, it was hypothesized that loss of the tracer is due to chemical interaction with the surface rather than due to mechanical trapping on rough surfaces.

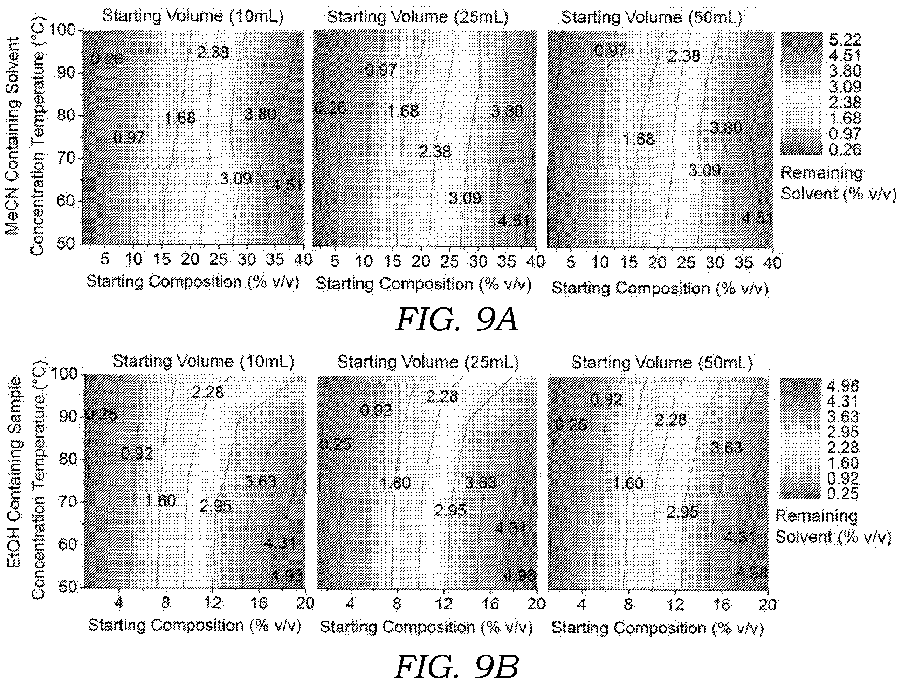

Even at the 100°C operating temperature, radio-HPLC analysis of tracers showed no significant difference in purity before and after concentration suggesting that tracers are stable under these conditions. Since high sample recovery was observed in all cases for one or more chip materials, one can also assume the tracers do not have significant volatility at 100°C. The degree of organic solvent removal during the concentration process was also examined. Because MeCN and EtOH are significantly more volatile than water (MeCN bp=82°C; EtOH bp= 78.37°C), it was suspected that these solvents would be preferentially lost compared to water. FIGS. 9A and 9B summarizes GC-MS measurements of the remaining organic solvent content after the concentration process as a function of starting solvent composition, evaporation temperature, and starting volume. Surprisingly, the starting sample volume (and hence total time to perform concentration process) has very minor effect on the final composition. It is suspected that the system rapidly reaches "equilibrium" where the majority of the chip 10 is filled with water (due to preferential removal of solvent), a small portion at the inlet is filled with the incoming sample solution, and a concentration gradient exists in between. Attainment of such a steady-state concentration gradient in the chip 10 is consistent with the results of the 50 mL evaporation experiments described above that showed no change in evaporation rate throughout the concentration process. Temperature had a small effect on final solvent composition, with higher temperature resulting in lower final solvent amount. The starting composition had the dominant effect on the final composition after concentration. The concentration procedure seems to reduce the initial organic solvent fraction roughly 10x for MeCN mixtures and roughly 5x for EtOH mixtures. Because the allowable injection limit for EtOH is 10% v/v, adequate EtOH removal will occur after concentration of samples with mobile phase composition up to 50% v/v EtOH; thus the chip 10 effectively performs formulation in addition to concentration. This encompasses the full range of EtOH-based mobile phases compatible with the microfluidic device 10. On the other hand, the allowed injection limit of MeCN is much lower (410 ppm or 0.041% v/v). Since typical HPLC mobile phases contain > 0.41% MeCN, the amount of residual MeCN will be too high for injection and an additional formulation process will be needed.

### Complete evaporation method

One way that the composition of MeCN could be reduced more than 10x would be to extend the "delay time," i.e., the additional evaporation time after the volume has already shrunk to the volume of the chip 10. Though the relationship between delay time and residual solvent amount was not investigated, the extreme case was studied where the mobile phase is completely evaporated, leaving only a dry residue of the PET tracer. To assess the ability to recover the tracer residue in an injectable solution such as saline, complete evaporation and recovery was characterized for two tracers (FIG. 8). In the case of [¹⁸F]Fallypride, the best recovery was achieved with the polyetherimide (Ultem™) chip (81.0 ± 6.3%, n=9). Recoveries in PMMA-machined and glass-PTFE were only 53.6 ± 28.6% (n=9) and 55.5 ± 20.7% (n=6), respectively. The low recovery and large variation suggests an adverse reaction of the tracer with the sample layer for these materials. For [¹⁸F]D-FEAU, PMMA-machined, glass-PTFE, and polyetherimide (Ultem™) all yielded high recovery (>93%) with glass-PTFE being the best (98.2 ± 0.9%, n=3). Recovery with PEEK was significantly worse and exhibited high variability (58.1 ± 12.1%, n=3), suggesting an adverse interaction. Since the performance appears to be tracer-specific, the ability to easily switch the sample layer material to suit the tracer is a major advantage of the design of the chip 10. Complete evaporation could be completed at a rate of ∼2 mL/min (depending on conditions) plus a 9.0 min "overhead" for sample loading, drying of solvent, collection of solute, and final rinse steps.

For three (3) samples of [¹⁸F]D-FEAU concentrated via the complete evaporation method, the residual MeCN concentrations in the first rinse measured via GC-MS were 700, 185, 87 ppm. Because, during operation, the initial rinse will be followed by two subsequent rinses, the final MeCN composition is therefore well below the allowed injection limit. As further assessment of tracer safety and stability, a [¹⁸F]D-FAC sample was injected into mice immediately after concentration via the partial evaporation method. No adverse reactions were observed in mice and the biodistribution was very similar to that from [¹⁸F]D-FAC injection obtained via conventional concentration and formulation with rotary evaporation. Furthermore, the biodistribution is similar to literature reports, showing the expected high uptake in immune-related organs such as the thymus, bone/bone marrow, and spleen.

In addition to solvents in the mobile phase itself, the purified HPLC fraction can also contain small amounts of the reaction solvent and it is important to consider whether these can be adequately removed, especially high-boiling (bp = 189°C) solvents such as DMSO. To answer this question, the evaporation rate of pure DMSO was first measured in the device at 100°C with the PEEK sample layer and found it to be 0.87 ± 0.03 mL/min (n = 5). This relatively fast rate suggested that adequate DMSO removal should be possible in a reasonable timeframe. Next, residual DMSO content was measured after performing microfluidic concentration. A 10 mL solution of DMSO (10,000 PPM in water) was concentrated using the complete evaporation method at 100°C using different delay times (270, 600, 870s) and then recovered with a water rinse. The normal delay time for complete evaporation (270s) resulted in a DMSO content of 1,400 PPM. Increased delay times of 600 and 870s resulted in remaining DMSO content of 800 PPM and 700 PPM, respectively. Operation at all delay times resulted in final compositions less than the injectable limit of 5000 PPM. Of course, the amount of solvent present in the purified fraction will depend on details of the purification protocol so measurements of residual solvents during synthesis optimization are recommended.

The automated system 60 that incorporates the microfluidic device 10 can be used concentrate and formulate various types of radiolabeled molecules such as PET tracers as well as prosthetic groups used to convert fragile biological molecules into PET tracers. It is especially useful for tracers intended for preclinical or *in vitro* applications where the needed radioactivity concentration is high but the total amount of radioactivity needed is low. The automated system, alternatively, could also be used to perform formulation for clinical applications.

The automated system 60 can be readily be integrated with upstream (e.g., purification) and downstream (e.g., sterile filtration) processes via tubing connections, and the operation is fully automated, ensuring straightforward operation and minimal operator exposure to radiation. The entire concentration and collection of a 10mL starting sample at 100°C can be completed in under nine (9) minutes for partial evaporation and under fourteen (14) minutes for complete evaporation. The small physical footprint of the complete system 60 allows for relatively compact and lightweight shielding, or frees up valuable space inside existing radiation-shielded hot cells that would otherwise be needed for concentration and formulation systems. This microfluidic approach is ideally suited for integration with either conventional radiosynthesizers lacking integrated or automated formulation systems, or for use in conjunction with emerging compact microscale radiosynthesizers. Because the vast majority of conventional synthesizers are focused on clinical production and do not include a means to concentrate the final tracer, this microfluidic concentration system could be used to extend their functionality to the production of tracers for preclinical imaging.

Depending on the architecture of the microfluidic chip 10, evaporation rates of up to 3.4 mL/min for water were observed at 100°C operating temperature. By using a serpentine channel with rounded corners, the system is able to achieve high tracer recovery (>97%) with the use of two rinse plugs, resulting in a total sample volume after concentration of ∼1.0-1.5 mL. The range of compatible solvents was also greatly expanded. At 100°C operating temperature, the system can handle samples with up to 80% (v/v) MeCN in H₂O, and up to 40% (v/v) EtOH in H₂O without significant loss of the sample. While these limits include the majority of mobile phases used in the production of PET tracers, these limits of organic solvent content could likely be further extended if needed, by decreasing operating temperatures (at the expense of evaporation rate) or by using a porous membrane 30 with even smaller pore 32 dimensions.

In partial evaporation mode, the amount of EtOH can be adequately reduced to safely allow direct injection after concentration (for mobile phases containing up to 50% EtOH v/v). To directly use samples containing MeCN, the complete evaporation method must be used to ensure the residual MeCN is below the allowed limit. Concentration to dryness could also be used if the mobile phase contained other toxic solvents that needed to be removed, such as DMSO. This ability to perform formulation at the same time as concentration significantly streamlines the overall PET tracer production process.

The ability to customize sample layer material was valuable for ensuring the highest possible performance. Several tracers exhibited significant (20-50%) losses when partnered with an unsuitable chip material, presumably due to adsorption or other adverse interactions. Materials with high thermal conductivity that are inert (or with protective inert coating formed thereon) may provide a way to achieve the highest possible evaporation speed combined with high sample recovery. For example, a polymer material with metal particles contained therein may provide high thermal conductivity with the inert or protective properties of a plastic-based material. In addition to the application described here, the microfluidic chip 10 could also be used in related areas such as the concentration of radioisotopes prior to labeling. For example, there is considerable interest in isotopes that can be produced via generators rather than cyclotrons (e.g., ^{6S}Ga, ⁸²Rb, and ⁶²Cu). However, a challenge of ⁶⁸Ga-labeling is that the output of the generator decreases over time, requiring larger and larger amounts of the eluent (HCl) to collect the desired amount of activity. While cartridge-based methods have been developed to concentrate gallium-68 into a consistent output volume, they are relatively slow, leading to considerable radioactive decay. The microfluidic device 10 may provide a means to more rapidly concentrate the isotope and achieve a similar result. In addition, concentration of radiometals can also help improve the reaction kinetics between the chelator and metal, and increase apparent specific activity by enabling reduced precursor usage. It has been observed that the concentration of [⁶⁸Ga]Ga³⁺ and [⁶⁴Cu]Cu²⁺ has significant effect on the kinetics and an automated means to concentrate these starting materials could lead to methods for improved labeling yields. More broadly, this system 60 can potentially be used for concentration or reformulation of any aqueous sample where compact size and high speed of liquid removal are important.

## Claims

1. A method of formulating or concentrating a radiolabeled molecule or compound comprising:
providing a microfluidic device (10) having a sample layer (12) containing a microfluidic channel (14) formed therein, a porous membrane (30) having a pore size of less than 0.5 µm disposed on the sample layer and covering the microfluidic channel, and a gas flow layer (40) having a gas-carrying channel (42) formed therein, wherein the porous membrane (30) is interposed between the sample layer (12) and the gas flow layer (40);
providing an automated system (60) for loading the microfluidic device with a sample fluid (40) contained in a sample reservoir (102) and a rinse solution (105) contained in a rinse solution reservoir (104), the automated system including a computer-controlled sample pressure regulator (76) coupled to a source of gas (78) for driving one or more of the sample fluid and rinse solution into the microfluidic device and a computer-controlled gas-flow pressure regulator (82) coupled to the source of gas for delivering the gas into the gas-carrying channel (42);
delivering the sample fluid containing the radiolabeled molecule or compound into the microfluidic channel using the gas from the computer-controlled sample pressure regulator;
heating the microfluidic device to at least partially evaporate the sample fluid in the microfluidic channel;
flowing the gas from the computer-controlled gas-flow pressure regulator through the gas-carrying channel to remove the evaporated fluid from the microfluidic device; and
applying a vacuum via the automated system to recover the radiolabeled molecule or compound into a collection reservoir (112).

2. The method of claim 1, wherein the porous membrane has a pore size of 0.2 µm or less.

3. The method of claim 1, wherein the microfluidic device further comprises a compression layer (50) disposed on the sample layer and comprising a plurality of fasteners (56) passing through the compression layer (50), sample layer (12), and gas flow layer (40).

4. The method of claim 1, wherein at least some of the sample fluid remains in the microfluidic channel during the evaporation operation.

5. The method of claim 1, wherein the sample fluid containing the radiolabeled molecule or compound is fully evaporated followed by the delivery of water, saline, or buffer.

6. The method of claim 1, wherein the sample fluid comprises one of ethanol (EtOH), methanol (MeOH), acetonitrile (MeCN), or dimethylsulfoxide (DMSO).

7. The method of claim 1, wherein the recovered radiolabeled molecule or compound is recovered in water or saline.

8. The method of claim 1, wherein the radiolabeled molecule or compound comprises an imaging tracer or a radiolabeled prosthetic group.

9. The method of claim 1, wherein the sample fluid is evaporated at a rate above 3.0 mL min⁻¹.

10. A system for formulating or concentrating a radiolabeled molecule or compound using a microfluidic device (10) comprising:
an automated system (60) for loading the microfluidic device (10) with a sample fluid (100) contained in a sample reservoir (102) and a rinse solution (105) contained in a rinse solution reservoir (104), the automated system including a computer-controlled sample pressure regulator(76) coupled to a source of gas (78) for driving one or more of the sample fluid and rinse solution into the microfluidic device, a computer-controlled gas-flow pressure regulator (82) coupled to the source of gas for delivering the gas into the gas-carrying channel, and a computer-controlled vacuum regulator (88) coupled to a source of vacuum (84) for removing one or more of the radiolabeled molecule or compound and a gas from the gas-carrying channel; and
the microfluidic device having a sample layer (12) containing a serpentine microfluidic channel (14) formed therein and having a width less than 3 mm, a porous membrane (30) having a pore size of less than 0.5 µm disposed on the sample layer and covering the serpentine microfluidic channel; and a gas flow layer (40) having a gas-carrying channel (42) formed therein and corresponding in shape to the serpentine microfluidic channel, wherein the porous membrane (30) is interposed and compressed between the sample layer (12) and the gas flow layer (40).

11. The system of claim 10, wherein the sample layer comprises a metallic sample layer.

12. The system of claim 10, wherein the serpentine microfluidic channel (14) comprises curved ends (16,18) between adjacent serpentine segments.

13. The system of claim 10, further comprising a compression layer (50) disposed on the sample layer and comprising a plurality of fasteners (56) passing through the compression layer (50), sample layer (12), and gas flow layer (40).

14. The system of claim 10, wherein the sample layer (12) comprises one of aluminum alloy, poly (methyl methacrvlate) (PMMA), polytetrafluoroethylene (PTFE), glass-filled PTFE (glass-PTFE), polyetherimide, cyclic olefin copolymer (COC), polyether ether ketone (PEEK), polyamide, polyamide-imide (PAI), poly-ether imide (PEI), polyphenylene sulfide (PPS), polybenzimidazole (PBI), thermoplastic polyimide (TPI), alloys of plastics, and glass.

## Patentansprüche

1. Verfahren zum Formulieren oder Konzentrieren eines radiomarkierten Moleküls
oder einer radiomarkierten Verbindung, umfassend:
Bereitstellen einer Mikrofluidikvorrichtung (10) mit einer Probenschicht (12), die einen darin ausgebildeten Mikrofluidikkanal (14) enthält, einer porösen Membran (30) mit einer Porengröße von weniger als 0,5 µm, die auf der Probenschicht angeordnet ist und den Mikrofluidikkanal bedeckt, und einer Gasströmungsschicht (40) mit einem darin ausgebildeten gasführenden Kanal (42), wobei die poröse Membran (30) zwischen der Probenschicht (12) und der Gasströmungsschicht (40) angeordnet ist;
Bereitstellen eines automatisierten Systems (60) zum Beladen der Mikrofluidikvorrichtung mit einem Probenfluid (40), das in einem Probenreservoir (102) enthalten ist, und einer Spüllösung (105), die in einem Spüllösungsreservoir (104) enthalten ist, wobei das automatisierte System aufweist: einen computergesteuerten Probendruckregler (76), der mit einer Gasquelle (78) gekoppelt ist, um eines oder mehrere von dem Probenfluid und der Spüllösung in die Mikrofluidikvorrichtung zu treiben, und einen computergesteuerten Gasströmungsdruckregler (82), der mit der Gasquelle gekoppelt ist, um das Gas in den gasführenden Kanal (42) einzuspeisen;
Einspeisen der Probenflüssigkeit, die das radioaktiv markierte Molekül oder die radiomarkierte Verbindung enthält, in den Mikrofluidikkanal unter Verwendung des Gases aus dem computergesteuerten Probendruckregler;
Erwärmen der Mikrofluidikvorrichtung, um das Probenfluid im Mikrofluidikkanal zumindest teilweise zu verdampfen;
Strömenlassen des Gases vom computergesteuerten Gasströmungsdruckregler durch den gasführenden Kanal, um das verdampfte Fluid aus der Mikrofluidikvorrichtung zu entfernen; und
Anlegen eines Vakuums über das automatisierte System zur Rückgewinnung des radioaktiv markierten Moleküls oder der Verbindung in einem Sammelreservoir (112).

2. Verfahren nach Anspruch 1, wobei die poröse Membran eine Porengröße von 0,2 µm oder weniger aufweist.

3. Verfahren nach Anspruch 1, wobei die Mikrofluidikvorrichtung ferner eine Kompressionsschicht (50) umfasst, die auf der Probenschicht angeordnet ist und eine Vielzahl von Befestigungselementen (56) umfasst, die durch die Kompressionsschicht (50), die Probenschicht (12) und die Gasströmungsschicht (40) hindurchgehen.

4. Verfahren nach Anspruch 1, wobei zumindest ein Teil des Probenfluids während des Verdampfungsvorgangs im Mikrofluidikkanal verbleibt.

5. Verfahren nach Anspruch 1, wobei das Probenfluid, welches das radioaktiv markierte Molekül oder die radiomarkierte Verbindung enthält, vollständig verdampft wird, gefolgt von der Einspeisung von Wasser, Kochsalzlösung oder Puffer.

6. Verfahren nach Anspruch 1, wobei das Probenfluid eines von Ethanol (EtOH), Methanol (MeOH), Acetonitril (MeCN) oder Dimethylsulfoxid (DMSO) umfasst.

7. Verfahren nach Anspruch 1, wobei das zurückgewonnene radioaktiv markierte Molekül oder die zurückgewonnene radiomarkierte Verbindung in Wasser oder Kochsalzlösung zurückgewonnen wird.

8. Verfahren nach Anspruch 1, wobei das radioaktiv markierte Molekül oder die radioaktiv markierte Verbindung einen Bildgebungs-Tracer oder eine radioaktiv markierte prothetische Gruppe umfasst.

9. Verfahren nach Anspruch 1, wobei das Probenfluid mit einer Geschwindigkeit von mehr als 3,0 ml min⁻¹ verdampft wird.

10. System zum Formulieren oder Konzentrieren eines radiomarkierten Moleküls oder einer radiomarkierten Verbindung unter Verwendung einer Mikrofluidikvorrichtung (10), umfassend:
ein automatisiertes System (60) zum Beladen der Mikrofluidikvorrichtung (10) mit einem Probenfluid (100), das in einem Probenreservoir (102) enthalten ist, und mit einer Spüllösung (105), die in einem Spüllösungsreservoir (104) enthalten ist, wobei das automatisierte System umfasst: einen computergesteuerten Probendruckregler (76), der mit einer Gasquelle (78) gekoppelt ist, um eines oder mehrere von dem Probenfluid und der Spüllösung in die Mikrofluidikvorrichtung zu treiben, einen computergesteuerten Gasströmungsdruckregler (82), der mit der Gasquelle gekoppelt ist, um das Gas in den gasführenden Kanal zu liefern, und einen computergesteuerten Vakuumregler (88), der mit einer Vakuumquelle (84) gekoppelt ist, um eines oder mehrere von dem radioaktiv markierten Molekül oder der Verbindung und ein Gas aus dem gasführenden Kanal zu entfernen; und
wobei die Mikrofluidikvorrichtung aufweist: eine Probenschicht (12), die einen darin ausgebildeten serpentinenartigen Mikrofluidikkanal (14) mit einer Breite von weniger als 3 mm, eine poröse Membran (30) mit einer Porengröße von weniger als 0,5 µm, die auf der Probenschicht angeordnet ist und den serpentinenartigen Mikrofluidikkanal bedeckt, enthält; und eine Gasströmungsschicht (40) mit einem darin ausgebildeten gasführenden Kanal (42), der in seiner Form dem serpentinenartigen Mikrofluidikkanal entspricht, wobei die poröse Membran (30) zwischen der Probenschicht (12) und der Gasströmungsschicht (40) angeordnet und komprimiert ist.

11. System nach Anspruch 10, wobei die Probenschicht eine metallische Probenschicht umfasst.

12. System nach Anspruch 10, wobei der serpentinenartige Mikrofluidikkanal (14) gekrümmte Enden (16, 18) zwischen benachbarten Serpentinensegmenten aufweist.

13. System nach Anspruch 10, ferner eine Kompressionsschicht (50) umfassend, die auf der Probenschicht angeordnet ist, und eine Vielzahl von Befestigungsmitteln (56) umfassend, die durch die Kompressionsschicht (50), die Probenschicht (12) und die Gasströmungsschicht (40) hindurchgehen.

14. System nach Anspruch 10, wobei die Probenschicht (12) eines umfasst von einer Aluminiumlegierung, Poly(methylmethacrylat) (PMMA), Polytetrafluorethylen (PTFE), glasgefülltem PTFE (Glas-PTFE), Polyetherimid, cyclischem Olefin-Copolymer (COC), Polyetheretherketon (PEEK), Polyamid, Polyamid-Imid (PAI), Polyether-Imid (PEI), Polyphenylensulfid (PPS), Polybenzimidazol (PBI), thermoplastischem Polyimid (TPI), Kunststofflegierungen, und Glas.

## Revendications

1. Procédé de formulation ou de concentration d'une molécule ou d'un composé radiomarqué, comprenant le fait de :
procurer un dispositif microfluidique (10) qui possède une couche (12) réservée à un échantillon, dans laquelle est réalisé un canal microfluidique (14), une membrane poreuse (30) possédant une dimension des pores qui est inférieure à 0,5 µm disposée sur la couche réservée à un échantillon et recouvrant le canal microfluidique, et une couche (40) réservée à un écoulement de gaz, dans laquelle est réalisé un canal (42) pour le transport du gaz ; dans lequel la membrane poreuse (30) est intercalée entre la couche (12) réservée à un échantillon et la couche (40) réservée à un écoulement de gaz ;
procurer un système automatisé (60) destiné au chargement du dispositif microfluidique avec un fluide (40) faisant office d'échantillon qui est contenu dans un réservoir (102) réservé à un échantillon et avec une solution de rinçage (105) qui est contenue dans un réservoir (104) réservé à une solution de rinçage, le système automatisé englobant un régulateur de la pression de l'échantillon (76) commandé par ordinateur, qui est couplé à une source de gaz (78) pour l'entraînement d'un ou de plusieurs éléments choisis parmi le fluide faisant office d'échantillon et la solution de rinçage jusque dans le dispositif microfluidique, ainsi qu'un régulateur de la pression de l'écoulement de gaz (82) commandé par ordinateur, qui est couplé à la source de gaz pour la distribution du gaz jusque dans le canal (42) pour le transport du gaz ;
distribuer le fluide faisant office d'échantillon qui contient la molécule ou le composé radiomarqué dans le canal microfluidique en utilisant le gaz à partir du régulateur de la pression de l'échantillon commandé par ordinateur ;
chauffer le dispositif microfluidique dans le but de faire en sorte que s'évapore au moins en partie le fluide faisant office d'échantillon dans le canal microfluidique ;
faire s'écouler le gaz à partir du régulateur de la pression de l'écoulement de gaz commandé par ordinateur à travers le canal de transport du gaz afin d'éliminer du dispositif microfluidique le fluide qui s'est évaporé ; et
appliquer un vide par l'intermédiaire du système automatisé dans le but de récupérer la molécule ou le composé radiomarqué dans un réservoir de récolte (112).

2. Procédé selon la revendication 1, dans lequel la membrane poreuse possède une dimension des pores de 0,2 µm ou moins.

3. Procédé selon la revendication 1, dans lequel le dispositif microfluidique comprend en outre une couche de compression (50) qui est disposée sur la couche réservée à un échantillon et qui comprend plusieurs dispositifs de fixation (56) qui traversent la couche de compression (50), la couche (12) réservée à un échantillon et la couche (40) réservée à un écoulement de gaz.

4. Procédé selon la revendication 1, dans lequel au moins une certaine quantité du fluide faisant office d'échantillon reste dans le canal microfluidique au cours de l'opération d'évaporation.

5. Procédé selon la revendication 1, dans lequel le fluide faisant office d'échantillon qui contient la molécule ou le composé radiomarqué fait l'objet d'une évaporation complète suivie de la distribution d'eau, d'une solution saline ou d'un tampon.

6. Procédé selon la revendication 1, dans lequel le fluide faisant office d'échantillon comprend un élément choisi parmi l'éthanol (EtOH), le méthanol (MeOH), l'acétonitrile (MeCN) ou le diméthylsulfoxyde (DMSO).

7. Procédé selon la revendication 1, dans lequel la molécule ou le composé radiomarqué recouvert est recouvert par de l'eau ou par une solution saline.

8. Procédé selon la revendication 1, dans lequel la molécule ou le composé radiomarqué comprend un traceur d'imagerie ou un groupe prothétique radiomarqué.

9. Procédé selon la revendication 1, dans lequel le fluide faisant office d'échantillon est évaporé à un débit supérieur à 3,0 ml min⁻¹.

10. Système destiné à la formulation ou à la concentration d'une molécule ou d'un composé radiomarqué en utilisant un dispositif microfluidique (10), comprenant :
un système automatisé (60) destiné au chargement du dispositif microfluidique (10) avec un fluide (100) faisant office d'échantillon qui est contenu dans un réservoir (102) réservé à un échantillon et avec une solution de rinçage (105) qui est contenue dans un réservoir (104) réservé à une solution de rinçage, le système automatisé englobant un régulateur de la pression de l'échantillon (76) commandé par ordinateur, qui est couplé à une source de gaz (78), pour l'entraînement d'un ou de plusieurs éléments choisis parmi le fluide faisant office d'échantillon et la solution de rinçage jusque dans le dispositif microfluidique, un régulateur de la pression du courant de gaz (82) commandé par ordinateur, qui est couplé à la source de gaz, pour la distribution du gaz jusque dans le canal de transport du gaz, et un régulateur de vide (88) commandée par ordinateur, qui est couplé à une source de vide (84) pour l'élimination d'un ou de plusieurs éléments choisis parmi la molécule ou le composé radiomarqué et un gaz à partir du canal de transport du gaz ; et
le dispositif microfluidique possédant une couche (12) réservée à un échantillon, dans laquelle est formé un canal microfluidique en serpentin (14) et qui possède une largeur inférieure à 3 mm, une membrane poreuse (30) possédant une dimension des pores qui est inférieure à 0,5 µm, disposée sur la couche réservée à un échantillon et recouvrant le canal microfluidique en serpentin ; et une couche (40) réservée à un écoulement de gaz, dans laquelle est réalisé un canal (42) pour le transport d'un gaz et qui correspond, en ce qui concerne sa configuration, à celle du canal microfluidique en serpentin ; dans lequel la membrane poreuse (30) est intercalée et est comprimé entre la couche (12) réservée à un échantillon et la couche (40) réservée à un écoulement de gaz.

11. Système selon la revendication 10, dans lequel la couche réservée un échantillon comprend une couche métallique réservée à un échantillon.

12. Système selon la revendication 10, dans lequel le canal microfluidique en serpentin (14) comprend des extrémités courbes (16, 18) entre des segments de serpentin adjacents.

13. Système selon la revendication 10, comprenant en outre une couche de compression (50) qui est disposée sur la couche réservée à un échantillon et qui comprend un certain nombre de dispositifs de fixation (56) qui traversent la couche de compression (50), la couche (12) réservée à un échantillon et la couche (40) réservée à un écoulement de gaz.

14. Système selon la revendication 10, dans lequel la couche (12) réservée à un échantillon comprend un élément choisi parmi un alliage à base d'aluminium, du polyméthacrylate de méthyle (PMMA), du polytétrafluoréthylène (PTFE), du PTFE contenant une matière de remplissage à base de verre (PTFE-verre), du polyéther imide, un copolymère oléfinique cyclique (COC), de la polyéther éther cétone (PEEK), du polyamide, du polyamide-imide (PAI), du polyéther imide (PEI), du polyphénylène sulfure (PPS), du polybenzimidazole (PBI), du polyimide thermoplastique (TPI), des alliages de matières plastiques et du verre.
